# EUROPEAN PATENT SPECIFICATION

(11) **EP 1 686 934 B1**
(45) Date of publication and mention of the grant of the patent: **18.03.2020**
(21) Application number: 04810414.5
(22) Date of filing: 04.11.2004
(51) Int. Cl.: A61L 27/12, A61L 27/50, A61F 2/46, A61B 17/56, A61F 2/28, A61F 2/44

(54) **INJECTABLE BONE SUBSTITUTE**
INJIZIERBARE KNOCHENSUBSTITUT
SUBSTITUT OSSEUX POUVANT ETRE INJECTE

(30) Priority: 07.11.2003 US 518475 P
(43) Date of publication of application: 09.08.2006
(73) Proprietor: Vivex Biologics Group, Inc., West Atlanta, GA 30339 (US)
(72) Inventor: LIN, Ruey-Mo, Tainan (CN); LIN, Jiin-Huey, Chern, Winnetka, IL 60093 (US); JU, Chien-Ping, Carbondale, IL 92901 (US)
(74) Representative: Gislon, Gabriele
(86) International application number: PCT/US2004/036951
(87) International publication number: WO 2005/046440

(56) References cited:
- WO-A2-02/34116
- WO-A2-98/16268
- US-A- 4 497 075
- US-A- 5 218 035
- US-A- 5 702 449
- US-A- 6 083 229

## Description

### FIELD OF THE INVENTION

The invention relates generally to an injectable bone substitute.

### BACKGROUND INFORMATION

A variety of interbody implants are available for spinal fusion procedures. These implants have been manufactured of various materials including steel, titanium, composites, allograft, xenograft or other biocompatible materials, and have the necessary strength to prevent the interbody space from collapsing before fusion has occurred. Other techniques for spinal fusion include the placement of bone graft material in the interbody space along with a plate or rod construct that spans the affected interbody space. Once fusion has occurred, the implants and hardware used to maintain the stability of the segment remain in the body to aid in stabilizing the spine.

Other types of implants have been developed from bio-compatible metals which incorporate threads on the outer surface of the implant that retain the implant in the interbody space after it is threaded therein. Still other implants have been developed that are made from bone. Examples of such spacers made from bone having use in spinal procedures are disclosed in U.S. Pat. No. 5,989,289. These spacers are provided with vertebral engaging surfaces on the upper and lower faces of the implant to resist migration of the implant in the interbody space and/or expulsion of the implant from the interbody space. While spacers made of bone can be readily incorporated in fusion procedures, the inherent brittle nature of bone resulting from a high mineral content, particularly load-bearing cortical bone, may limit its potential for use in applications that require the implant to resist loading. For example, cortical bone typically consists of approximately 70% mineral content and 30% non-mineral matter. Of this non-mineral matter, approximately 95% is type I collagen, with the balance being cellular matter and non-collagenous proteins.

WO98/16268 A2 discloses a bone substitute wherein a bioresorbable PCA calcium phosphate, formulated for rapid bioresorbability, is used. An implant is prepared as an injectable paste from said calcium phosphate in order to join and hold bone fragments in place or to improve the adhesion of a hip prosthesis. In particular, the calcium phosphate is pre-formed into a disk or shim and is a slowly resorbable spacer to be implanted. In one embodiment the disk is a hollow ring and said PCA calcium phosphate is filled in the center of said ring.

The procedure used for fusing both the posterior and anterior elements of an unstable spinal location simultaneously is known as a 360° fusion. The most common method used for 360° fusion, following preparation of the interbody space, as needed, is to use metallic implants such as rods and screws, or plates and screws to fix the posterior elements of the interbody space. The anterior elements are typically fused using either solid allograft/autograft bone dowels/plugs (cortical and cancellous components), or a metallic/carbon "cage" implant filled with autograft, allograft or a bone substitute material.

Bone grafts have commonly been used in a fixed shape, pulverized, or as pliable demineralized bone. One form of a pliable bone graft is a demineralized bone material typically in the form of a sponge or putty having very little structural integrity. While a demineralized bone segment may retain properties suitable to support bone ingrowth, the structural properties of the bone are altered by removal of its mineral content. Thus, such bone sponges and putties may not typically be used in load-bearing applications without assistance from a plate or rod construct that spans the affected interbody space.

Therefore, there remains a need for new methods for performing spinal fusion that result in implants having the requisite load carrying capabilities.

### SUMMARY OF THE INVENTION

The invention is based on the discovery that an injectable calcium phosphate-based bone substitute can be used to perform single, or multi level spinal fusions, such as 360° spinal fusions used in treatment of degenerative disc disease.

In one embodiment, the invention provides a bone substitute for spinal fusion in a subject, the bone substitute comprising calcium phosphate, which bone substitute is injectable into one or more interbody spaces in the subject by injection through a syringe, catheter, or cannula to facilitate single, or multi level spinal fusion in a segment of the spine selected from cervical, thoracic, lumbar, lumbosacral and SI joint, and combinations thereof, wherein the calcium phosphate in the bone substitute essentially consists of monolithic tetracalcium phosphate (Ca4(PO4)2O).

Disclosed (not claimed) are also methods for performing one or more spinal fusions in a subject comprise introducing an effective amount of an injectable calcium phosphate-based bone substitute into one or more interbody spaces in the subject by injection through a syringe, catheter, or cannula to facilitate single, or multi level spinal fusion.

Further disclosed (not claimed) is also a method for performing one or more spinal fusions on a subject include placing in the posterior portion of at least one suitable interbody space a metallic implant selected from rods and pedicle screws or plates and pedicle screws by attachment to adjacent vertebrae. An effective amount of a calcium phosphate-based bone substitute is injected into the anterior portion of the interbody; and allowed to solidify in vivo, thereby performing the spinal fusion.

### DETAILED DESCRIPTION OF THE INVENTION

The present invention is based on the discovery that an injectable calcium phosphate-based bone substitute can be used to facilitate bone fusion, such as 360° spinal fusion.

### Patient Preparation

The subject is placed in the prone position on the operating table. The subject is then prepped and draped to allow surgical access to diseased spine level(s). General anesthesia is administered. Surgical approach to targeted spine motion segment level(s) and incision site are confirmed by x-ray.

### Surgical Approach

Using a posterior midline incision over the diseased motion segment(s), tissue is dissected to expose the spinous process and lamina of the targeted posterior spine level(s). Any pathology causing symptomatic claudication of the posterior spine neural anatomy is removed. Removal of suspect tissue may require a laminectomy, laminotomy or foramenotomy and resection/dissection of the ligamentum flavum. It is possible that neural compression is transient, resulting from an unstable motion segment. Instability can be caused by the collapse of the interbody space due to disc degeneration. In this case, it may not be necessary to decompress tissue, but only to stabilize the motion segment(s).

Once the decompression is complete, the motion segment may need to be mechanically stabilized. Instability is checked by manipulation of the spine. Even if instability exists with no decompression, the spine will still need to be stabilized.

Disc degeneration is now commonly treated with a 360° motion segment fusion, whereby both the anterior and posterior spine elements of the interbody space are fused. It is important to consider the mechanical stresses place on the anterior and posterior elements when considering a fusion technique. The anterior motion segment elements (vertebral bodies and disc) bear approximately 80% of the compressive force at that given level in the spine. The posterior 1/3 of the vertebral body and disc represent the center point for axial compression in the spine. These mechanics are critical for assessing what type of fusion will have the best clinical outcome for a given pathology. In cases where a 360° fusion procedure is deemed the best technique, the invention provides a spinal fusion procedure in which an injectable bone substitute with a suitable compressive strength profile is used for the anterior portion of the fusion.

### 360° Fusion Technique

In one embodiment, not according to the invention, methods for performing one or more spinal fusions in a subject include introducing an effective amount of an injectable calcium phosphate-based bone substitute into one or more suitable interbody spaces in the subject by injection through a syringe, catheter, or cannula to facilitate single, or multi level spinal fusion. The bone substitute is allowed to set under physiological conditions, i.e., in vivo, over time. Preferably, the bone substitute sets by hardening to form a solid mass and allows ingrowth of autologous bone in vivo over time. The methods for fixation of the interbody space in its posterior portion can be any method known in the art. The new aspects of the invention methods reside in the technique applied to the anterior portion of the fusion as well as in the combination of known standard posterior fixation techniques with the new methods for using an injectable calcium phosphate-based bone substitute to fill the anterior interbody space.

In another embodiment, not according to the present invention, methods for performing one or more spinal fusion on a subject comprise placing in the posterior portion of at least one suitable interbody space a metallic implant selected from rods and pedicle screws or plates and pedicle screws by attachment thereof to adjacent vertebrae; injecting into the anterior portion of the interbody space an effective amount of a calcium phosphate-based bone substitute; and allowing the bone substitute to set in vivo.

The methods not according to the present invention for performing spinal fusions can be performed by using either an anterior, posterior, or posterolateral approach to the interbody space. The posterolateral approach (unilateral or bilateral) reduces surgical morbidity over an anterior approach, but caution is required while working around the *cauda equina* and exiting nerve roots in the spinal canal. Posterior access and visualization of the interbody space is more limited than with the anterior approach, but many spinal surgeons are trained in how to deal with those circumstances.

The anterior approach for the anterior portion of the 360° fusion can be done with an open retro-peritoneal technique, or endoscopically. Although approaching the spine anteriorly can lead to a higher risk of complications and more blood loss, visualization and disc access is greatly improved over a posterior technique.

The surgical site(s) can be closed using standard suturing techniques.

A "suitable interbody space" as the term is used in the application and claims herein means the space between adjacent vertebrae where a disc resides in a healthy spine but which is either at least partially devoid of disc material due to wear and tear on the vertebral column or has been prepared using one or a combination of the above techniques, as are known in the art, to surgically create a void in the disc space.

For example, the interbody space can be prepared, as needed, by combining a nuclectomy with denuding of the caudal and cephalad vertebral end plates. Denuding the cartilaginous end-plates enables direct bone to bone substitute material contact, which is critical for bone fusion. A bilateral posterolateral approach (versus unilateral) may be needed for adequate interbody space preparation in certain posterior approach cases. In any event, preparation of the interbody space can comprise one or more techniques selected from annulatomy, nuclectomy, denuded end-plates; decorticated end-plates; and intradiscal electrothermal treatment.

For preparation of the posterior portion of the interbody space, the posterolateral gutter is decorticated and covered by bone and/or a bone substitute. Posterior instrumentation can then by applied to the spine utilizing plates or rods secured by pedicle screws to adjacent vertebral bodies.

A "subject" as the term is used herein is any mammal, including zoo, farm and domestic animals and humans.

An "effective amount" of the injectable calcium-phosphate-based bone substitute as the term is used herein is an amount effective to accomplish fusion of vertebrae adjacent to the interbody site in the subject.

"Setting time" as the term is used herein is the time after which a 1 mm diameter pin with a load of 1/4 pound (1,11 N)) can be inserted only 1 mm deep into the surface of a CPC paste, as determined using ISO 1566, a method commonly used for measuring the setting time of dental zinc phosphate cements as well as CPC.

"Working time" as the term is used herein means the time after which a CPC paste becomes too viscous to be stirred. Generally working time is a few minutes shorter than setting time.

After setting for about 30 minutes, the bone substitute suitable for use in the invention methods has a minimum compressive strength of about 10 MPa and a minimum compressive strength of 25 MPa is obtained within 24 hours after injection or after exposure to physiological conditions. The compressive strength herein is as determined using ASTM F451-99, a method that is commonly used for the compressive strength measurement of CPC.

The injectable calcium phosphate-based bone substitute is introduced into the anterior portion of the interbody space in the disclosed methods using a syringe, catheter, cannula, or the like. An injectable calcium phosphate-based bone substitute suitable for use in the disclosed methods will have viscosity capable of flowing through a 24 gauge needle, or larger, and working and setting times of about 5 to about 30 minutes. After setting for about 30 minutes, the suitable bone substitute has a minimum compressive strength of about 10 MPa, or a minimum of 25 MPa compressive strength within 24 hours after injection. Additionally, when solidified, the bone substitute can have a porosity of about 20% to about 50% by volume as measured using ASTM C830-00 water saturation technique.

The injectable calcium phosphate-based bone substitute having these characteristics can consist essentially of calcium phosphate, for example being a substantially monolithic tetracalcium phosphate (Ca₄(PO₄)₂O). The calcium phosphate may further comprise surface whiskers or fine needles of calcium phosphate, said whiskers having a length up to about 5000 nm and a width up to about 500 nm, for example, a length from about 1nm to about 2000 nm and a width from about 1 nm to about 200 nm. Alternatively, the suitable calcium phosphate-based bone substitute can comprise minor amounts of additional substances, such as Na₃PO_{4;} Na₂HPO_{4;} NaH₂PO₄; Na₄HPO₄.7H₂O; Na₃PO₄.12H₂O; H₃PO_{4;} CaSO_{4;} (NH₄) ₃PO_{4;} (NH₄)₂HPO_{4;} (NH₄)H₂PO₄; (NH₄)₃PO₄.3H₂O; NaHCO₃; CaCO₃; Na₂CO₃; KH₂PO₄; K₂HPO₄; K₃PO₄; CaF₂: SrF₂; Na₂SiF₆; Na₂PO₃F, and the like. The suitable bone substitute can also comprise an amount of one or more active agents suitable to promote bone growth, such as a growth factor, a bone morphology protein, or a pharmaceutical carrier therefor.

Examples of suitable calcium phosphates that can be used in preparation of the injectable calcium phosphate-based bone substitutes used in the disclosed methods include, but are not limited to, Ca₄(PO₄)2O, CaHPO₄.2H2O, CaHPO₄, Ca₈H₂(PO₄)₆.5H₂O, alpha-Ca3(PO₄)₂, beta-Ca3(PO₄)₂, Ca₂P₂O₇, Ca₂H₂P₂O₈, and the like.

Calcium-phosphate-based cements and bone substitutes suitable for use in the disclosed methods, and methods for their preparation, are described, for example in U.S. Patent Nos. 6,379,453 B1 and 6,616,742 and in co-pending U.S. patent application Serial Nos. 09/351,912, filed July 14, 1999; 09/941,576, filed August 30, 2001; 10/179,879, filed June 26, 2002; and 10/328,019, filed December 26, 2002.

Although the invention has been described with respect to specific embodiments, it will be understood that modifications and variations are encompassed within the scope of the invention. Accordingly, the invention is limited only by the following claims.

## Claims

1. A bone substitute for spinal fusion in a subject, the bone substitute comprising calcium phosphate, which bone substitute is injectable into one or more interbody spaces in the subject by injection through a syringe, catheter, or cannula to facilitate single, or multi level spinal fusion in a segment of the spine selected from cervical, thoracic, lumbar, lumbosacral and SI joint, and combinations thereof, wherein the calcium phosphate in the bone substitute essentially consists of monolithic tetracalcium phosphate (Ca₄(PO₄)₂O).

2. The bone substitute according to claims 1 or 2 wherein the bone substitute is injectable into the one or more interbody spaces by an approach selected from posterior, posterolateral, anterior, anterolateral and lateral approaches, and combinations thereof.

3. The bone substitute according to any of the preceding claims wherein the bone substitute is transformable from a viscous consistency to a solid consistency over time.

4. The bone substitute according to any of the preceding claims having a minimum compression strength of 10 MPa after setting for about 30 minutes and preferable is solidifiable to a compression strength of 25 MPa within 24 hours of injection.

5. The bone substitute according to any of the preceding claims having a viscosity capable of flowing through at least a 24 gauge needle.

6. The bone substitute according to any of the preceding claims having a porosity of about 20% to about 50% by volume, on solidification.

7. The bone substitute according to any of the preceding claims further comprising one or more of the following: Na₃PO₄; Na₂HPO_{4;} NaH₂PO₄; Na₄HPO₄.7H₂O; Na₃PO₄.12H₂O; H₃PO₄; CaSO₄; (NH₄)₃PO₄; (NH₄)₂HPO₄; (NH₄)H₂PO₄; (NH₄)₃PO₄.3H₂O; NaHCO₃; CaCO₃; Na₂CO₃; KH₂PO₄; K₂HPO₄; K₃PO₄; CaF₂; SrF₂; Na₂SiF₆; Na₂PO₃F.

8. The bone substitute according to any of the preceding claims further comprising one or more of the following one or more active ingredients suitable to promote bone growth, such as a growth factor, a bone morphology protein, or a pharmaceutical carrier therefor.

9. The bone substitute according to any of the preceding claims further comprising surface whiskers or fine needles of calcium phosphate, said whiskers preferably having a length up to about 5000 nm and a width up to about 500 nm, and more preferably a length from about 1nm to about 2000 nm and a width from about 1nm to about 200nm.

10. The bone substitute according to any of the preceding claims suitable for facilitating the anterior fusion of vertebrae without the use of pre-formed interbody spacers, cages, dowels or plugs consisting of a biologic or non-biologic material and/or without posterior spine fixation.

11. The bone substitute according to any of the preceding claims bone being bioresorbable, allowing ingrowth of autologous bone during resorption.

12. The bone substitute according to any of the preceding claims wherein the injectable bone substitute is settable in an interbody space and is maintainable in the body as a solid for an extended period of time, preferably up to and including the duration of the life of the subject.

13. Use of a monolithic tetracalcium phosphate (Ca₄(PO₄)₂O), for the preparation of a bone substitute according to any claim 1 to 12, wherein said bone substitute may further comprise one or more of CaHPO₄.2H₂O, CaHPO₄, Ca₈H₂(PO₄)₆.5H₂O, alpha-Ca₃(PO₄)₂, beta-Ca₃(PO₄)₂, Ca₂P₂O₇, Ca₂H₂P₂O₈.

14. Method for preparing a bone substitute according to any of the preceding claims 1-12 for injection into a subject, comprising the step of introducing the injectable bone substitute into a medical instrument, prior to injection into the subject, the medical instrument preferably being a syringe, catheter or cannula.

15. A device, selected from a syringe, catheter or cannula, containing the injectable bone substitute according to any one of claims 1-12.

## Patentansprüche

1. Knochenersatzmittel für Wirbelsäulenfusionen in einem Patienten, wobei das Knochenersatzmittel Calciumphosphat umfasst, wobei das Knochenersatzmittel in einen oder mehrere Zwischenkörperräume in dem Patienten durch Injektion durch eine Spritze, einen Katheter oder eine Kanüle injizierbar ist, um eine Einzelebenen- oder Mehrebenen-Wirbelsäulenfusion in einem Segment der Wirbelsäule zu ermöglichen, das aus dem Hals-, Brust-, Lenden-, Lumbosakral- und SI-Gelenk und Kombinationen davon ausgewählt ist, wobei das Calciumphosphat in dem Knochenersatzmittel im Wesentlichen aus monolithischem Tetracalciumphosphat (Ca₄(PO₄)₂O) besteht.

2. Knochenersatzmittel nach einem der Ansprüche 1 oder 2, wobei das Knochenersatzmittel in den einen oder die mehreren Zwischenkörperräume durch einen Ansatz injizierbar ist, der aus posterioren, posterolateralen, anterioren, anterolateralen und lateralen Ansätzen und Kombinationen davon ausgewählt ist.

3. Knochenersatzmittel nach einem der vorherigen Ansprüche, wobei sich das Knochenersatzmittel mit der Zeit von einer viskosen Konsistenz in eine feste Konsistenz umwandelt.

4. Knochenersatzmittel nach einem der vorherigen Ansprüche, wobei das Knochenersatzmittel eine minimale Druckfestigkeit von 10 MPa nach Setzen für etwa 30 Minuten aufweist, und vorzugsweise bis zu einer Druckfestigkeit von 25 MPa innerhalb eines Zeitraums von 24 Stunden nach Injektion verfestigbar ist.

5. Knochenersatzmittel nach einem der vorherigen Ansprüche, wobei das Knochenersatzmittel eine Viskosität aufweist, die dazu geeignet ist, durch mindestens eine 24 Gauge-Nadel zu fließen.

6. Knochenersatzmittel nach einem der vorherigen Ansprüche, wobei das Knochenersatzmittel beim Verfestigen eine Porosität von etwa 20 bis etwa 50 Vol.-% aufweist.

7. Knochenersatzmittel nach einem der vorherigen Ansprüche, weiterhin umfassend eine oder mehrere der Folgenden: Na₃PO₄, Na₂HPO₄, NaH₂PO₄, Na₄HPO₄.7H₂O, Na₃PO₄.12H₂O, H₃PO₄, CaSO₄, (NH₄)₃PO₄, (NH₄)₂HPO₄, (NH₄)H₂PO₄, (NH₄)₃PO₄.3H₂O, NaHCO₃, CaCO₃, Na₂CO₃, KH₂PO₄, K₂HPO₄, K₃PO₄, CaF₂, SrF₂, Na₂SiF₆, Na₂PO₃F.

8. Knochenersatzmittel nach einem der vorherigen Ansprüche, weiterhin umfassend ein oder mehrere der folgenden einen oder mehreren aktiven Bestandteile, die geeignet sind, das Knochenwachstum zu fördern, wie beispielsweise Wachstumsfaktoren, ein Knochenmorphologieprotein oder ein pharmazeutischer Träger davon.

9. Knochenersatzmittel nach einem der vorherigen Ansprüche, weiterhin umfassend Oberflächenfadenkristalle oder feine Nadeln von Calciumphosphat, wobei die Fadenkristalle vorzugsweise eine Länge von ungefähr 5000 nm und eine Weite von ungefähr 500 nm, und vorzugsweise eine Länge von ungefähr 1 nm bis ungefähr 2000 nm und eine Weite von ungefähr 1 nm bis ungefähr 200 nm aufweisen.

10. Knochenersatzmittel nach einem der vorherigen Ansprüche, geeignet für die Erleichterung der anterioren Fusion von Wirbeln ohne die Verwendung von vorgeformten Zwischenkörper-Abstandhaltern, Käfigen, Dübeln oder Stopfen, bestehend aus einem biologischen oder nicht biologischen Material, und/oder ohne eine posteriore Wirbelsäulenbefestigung.

11. Knochenersatzmittel nach einem der vorherigen Ansprüche, wobei das Knochenersatzmittel bioresorbierbar ist, wodurch das Einwachsen von autologem Knochen während der Resorption ermöglicht wird.

12. Knochenersatzmittel nach einem der vorherigen Ansprüche, wobei das injizierbare Knochenersatzmittel in einen Zwischenkörperraum setzbar ist und über einen längeren Zeitraum als Feststoff im Körper gehalten wird, wobei der verlängerte Zeitraum vorzugsweise bis zu und einschließlich der Dauer des Lebens des Subjekts beträgt.

13. Verwendung eines monotlithischen Tetracalciumphosphates (Ca₄(PO₄)₂O) für die Herstellung eines Knochenersatzmittels nach einem der Ansprüche 1 bis 12, wobei das Knochenersatzmittel weiterhin eine oder mehrere det Folgenden umfassen kann: CaHPO₄.2H₂O, CaHPO₄, Ca₈H₂(PO₄)₆.5H₂O, alpha-Ca₃(PO₄)₂, beta-Ca₃(PO₄)₂, Ca₂P₂O₇, Ca₂H₂P₂O₈.

14. Verfahren zur Herstellung eines Knochenersatzmittels nach einem der vorherigen Ansprüche 1 bis 12 zur Injektion in einen Patienten, umfassend den Schritt des Einführens des injizierbaren Knochenersatzmittels in ein medizinisches Gerät vor der Injektion in einen Patienten, wobei das medizinische Gerät vorzugsweise eine Spritze, ein Katheter oder eine Kanüle ist.

15. Vorrichtung, ausgewählt aus einer Spritze, einem Katheter oder einer Kanüle, enthaltend das injizierbare Knochenersatzmittel nach einem der Ansprüche 1 bis 12.

## Revendications

1. Substitut osseux pour arthrodèse chez un sujet, le substitut osseux comprenant du phosphate de calcium, lequel substitut osseux est injectable dans un ou plusieurs espaces de corps intermédiaires chez le sujet par injection via une seringue, un cathéter ou une canule pour faciliter une arthrodèse à un niveau unique ou à de multiples niveaux dans un segment de la colonne vertébrale choisi parmi un joint cervical, thoracique, lombaire, lombo-sacré et SI et leurs combinaisons, dans lequel le phosphate de calcium dans le substitut osseux consiste essentiellement en des phosphate tétracalcique monolithique (Ca₄(PO₄)₂O).

2. Substitut osseux selon les revendications 1 ou 2, dans lequel le substitut osseux est injectable dans les un ou plusieurs espaces de corps intermédiaires par une approche choisie parmi les approches postérieure, postérolatérale, antérieure, antérolatérale et latérale et leurs combinaisons.

3. Substitut osseux selon l'une quelconque des revendications précédentes, dans lequel le substitut osseux est transformable d'une consistance visqueuse à une consistance solide au fil du temps.

4. Substitut osseux selon l'une quelconque des revendications précédentes, ayant une résistance à la compression minimale de 10 MPa après figeage pendant environ 30 minutes et de préférence étant solidifiable à une résistance à la compression de 25 MPa dans les 24 heures d'injection.

5. Substitut osseux selon l'une quelconque des revendications précédentes, ayant une viscosité capable de permettre un écoulement à travers au moins une aiguille de jauge de 24.

6. Substitut osseux selon l'une quelconque des revendications précédentes, ayant une porosité d'environ 20 % à environ 50 % en volume lors de la solidification.

7. Substitut osseux selon l'une quelconque des revendications précédentes, comprenant en outre un ou plusieurs des suivants: Na₃PO₄; Na₂HPO₄; NaH₂PO₄; Na₄HPO₄.7H₂O; Na₃PO₄.12H₂O; H₃PO₄; CaSO₄; (NH₄)₃PO₄; (NH₄)₂HPO₄; (NH₄)H₂PO₄; (NH₄)₃PO₄.3H₂O; NaHCO₃; CaCO₃; Na₂CO₃; KH₂PO₄; K₂HPO₄; K₃PO₄; CaF₂; SrF₂; Na₂SiF₆; Na₂PO₃F.

8. Substitut osseux selon l'une quelconque des revendications précédentes, comprenant en outre un ou plusieurs des un ou plusieurs ingrédients actifs suivants convenant pour promouvoir une croissance osseuse, notamment un facteur de croissance, une protéine de morphologie osseuse ou un véhicule pharmaceutique pour celle-ci.

9. Substitut osseux selon l'une quelconque des revendications précédentes, comprenant en outre des barbes de surface ou de fines aiguilles de phosphate de calcium, lesdites barbes ayant de préférence une longueur montant jusqu'à environ 5 000 nm et une largeur montant jusqu'à environ 500 nm, et plus préférentiellement une longueur d'environ 1 nm à environ 2 000 nm et une largeur d'environ 1 nm à environ 200 nm.

10. Substitut osseux selon l'une quelconque des revendications précédentes convenant pour faciliter la fusion antérieure de vertèbres sans l'utilisation d'espaceurs, de cages, de chevilles ou de bouchons de corps intermédiaires préformés consistant en un matériau biologique ou non biologique et/ou sans fixation de colonne vertébrale postérieure.

11. Substitut osseux selon l'une quelconque des revendications précédentes, l'os étant biorésorbable, permettant la croissance d'os autologue au cours de la résorption.

12. Substitut osseux selon l'une quelconque des revendications précédentes, dans lequel le substitut osseux injectable peut être figé dans un espace de corps intermédiaire et peut être maintenu dans le corps sous une forme solide pendant une période de temps prolongée, de préférence jusqu'à et y compris la durée de la vie du sujet.

13. Utilisation d'un phosphate tétracalcique monolithique (Ca₄(PO₄)₂O) pour la préparation d'un substitut osseux selon l'une quelconque des revendications 1 à 12, dans laquelle ledit substitut osseux peut comprendre en outre un ou plusieurs parmi CaHPO₄.2H₂O, CaHPO₄, Ca₈H₂(PO₄)₆.5H₂O, alpha-Ca₃(PO₄)₂, bêta-Ca₃(PO₄)₂, Ca₂P₂O₇, Ca₂H₂P₂O₈.

14. Procédé de préparation d'un substitut osseux selon l'une quelconque des revendications précédentes 1 à 12 pour injection chez un sujet, comprenant l'étape d'introduction du substitut osseux injectable dans un instrument médical, avant injection chez le sujet, l'instrument médical étant de préférence une seringue, un cathéter ou une canule.

15. Dispositif choisi parmi une seringue, un cathéter ou une canule, contenant le substitut osseux injectable selon l'une quelconque des revendications 1 à 12.
